(19) Europäisches Patentamt
European Patent Office
Office européen des brevets

(11) Veröffentlichungsnummer: **0 168 733**
**A2**

(12) **EUROPÄISCHE PATENTANMELDUNG**

(21) Anmeldenummer: **85108398.0**

(22) Anmeldetag: **06.07.85**

(51) Int. Cl.⁴: **C 07 D 215/56**, C 07 D 215/58, C 07 D 471/04 // (C07D471/04, 221:00, 221:00)

(30) Priorität: **18.07.84 DE 3426486**

(43) Veröffentlichungstag der Anmeldung: **22.01.86 Patentblatt 86/4**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Grohe, Klaus, Dr., Am Wasserturm 10, D-5068 Odenthal (DE)**

(54) **Verfahren zur Herstellung von Chinolon- und Naphthyridoncarbonsäuren.**

(57) Verfahren zur Herstellung von Chinolon- und Naphthyridoncarbonsäuren der Formel

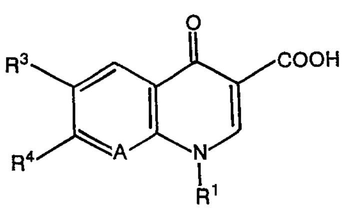

durch Cyclokondensierung entsprechender Aminoacrylsäurederivate und anschließende Verseifung. Die Verbindungen sind wertvolle Zwischenprodukte für die Herstellung antibakterieller Substanzen.

EP 0 168 733 A2

0168733

BAYER AKTIENGESELLSCHAFT 5090 Leverkusen, Bayerwerk
Konzernverwaltung RP
Patentabteilung Si/ABc

# Verfahren zur Herstellung von Chinolon- und Naphthyridon-carbonsäuren

Die vorliegende Erfindung betrifft ein Verfahren zur Herstellung von Chinolon- und Naphtyridoncarbonsäuren, die wertvolle Zwischenprodukte für die Synthese hochwirksamer antibakterieller Arzneimittel sind.

Gegenstand der Erfindung ist ein Verfahren zur Herstellung von Chinolon- und Naphthyridon-3-carbonsäuren der Formel I, das dadurch gekennzeichnet ist, daß man Amino-acrylsäurederivate der Formel III, in der

X die Nitrogruppe, eine Alkoxy-, Alkylmercapto- oder Alkylsulfonylgruppe mit jeweils bis zu drei Kohlenstoffatomen sein kann,

$R^1$ $C_1$-$C_3$-Alkyl, Cyclopropyl, Phenyl, die Amino-, Methylamino- oder Dimethylaminogruppe darstellt,

$R^2$ eine Alkoxycarbonylgruppe mit 1-3 Kohlenstoffatomen im Alkoholteil bedeutet,

$R^3$ Wasserstoff, Nitro oder Halogen, insbesondere Fluor oder Chlor, bedeutet,

$R^4$ Wasserstoff oder Halogen, insbesondere Fluor oder Chlor, darstellt, und

A Stickstoff oder ein gegebenenfalls substituiertes Kohlenstoffatom C-$R^5$ sein kann, wobei

$R^5$ Wasserstoff, Fluor oder Chlor bedeutet,

zur Verbindung der Formel II cyclokondensiert und die Alkoxycarbonylgruppe $R^2$ zur Carboxylgruppe verseift.

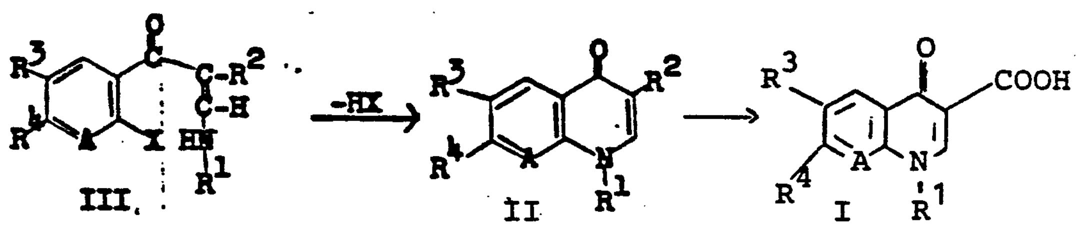


Aus der Europäischen Patentanmeldung 0 004 279 ist zwar eine Cyclisierung von Aminoacrylsäurederivaten der Formel IV zu Chinolon- bzw. Azachinoloncarbonsäuren der Formel V bekannt, wobei $X^1$ Halogen bedeutet:

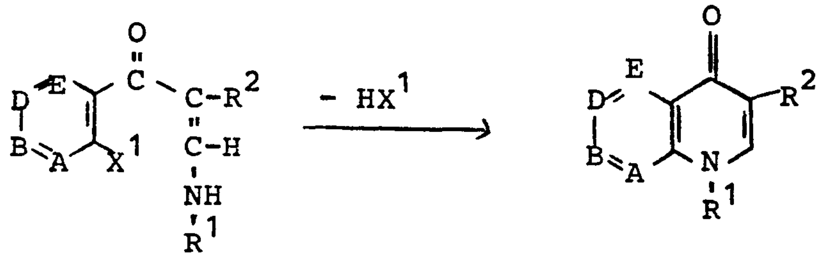


IV V

Es ist jedoch als ausgesprochen überraschend zu bezeichnen, daß die erfindungsgemäße Cyclokondensation von III zu II unter Abspaltung von Alkohol, Alkylmercaptan, salpetriger Säure oder Alkansulfinsäure verläuft.

Die Cyclisierungsreaktion III ⟶ II wird in einem Temperaturbereich von etwa 60-300°C, bevorzugt 80-180°C durchgeführt.

Als Verdünnungsmittel können Dioxan, Dimethylsulfoxid, N-Methylpyrrolidon, Sulfolan und bevorzugt N, N-Dimethylformamid verwendet werden.

Als Kondensationsmittel kommen für diese Reaktionsstufe Natrium- oder Kaliumalkoholate mit 1-4 C-Atomen im Alkoholrest, z.B. Kalium-tert.-butanolat oder Natriummethylat sowie Natriumhydrid und besonders Natrium- oder Kaliumcarbonat in Betracht.

Die im letzten Schritt erfolgende Esterhydrolyse von II ($R^2$ = COOAlkyl) unter basischen oder sauren Bedingungen führt zu den Chinolon- bzw. Azachinoloncarbonsäuren I.

Verwendet man bei der Cyclokondensation von III zu II beispielsweise den (5-Chlor-2,4-dinitro-benzoyl)-3-cyclopropylaminoacrylsäureethylester, so kann der Reaktionsverlauf durch das folgende Formelschema wiedergegeben werden:

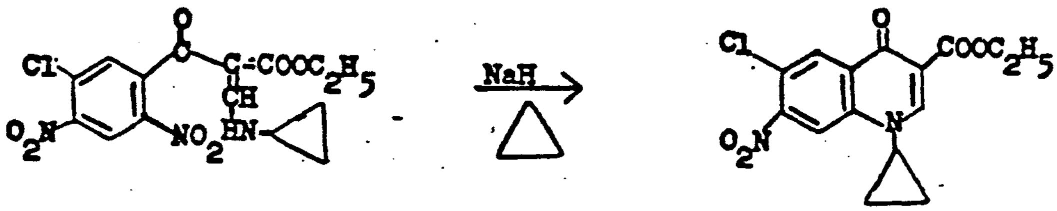


Falls $R^4$ der Verbindungen II Halogen darstellt, kann dieses durch Umsetzung mit Aminen gegen den Aminrest ausgetauscht werden. Die gebildeten 7-Amino-chinolon- und 7-Aminonaphthyridoncarbonsäuren sind hochwirksame Bakterizide. So erhält man beispielsweise durch Umsetzung von IIa ($R^1$ = Cyclopropyl, $R^2$ = COOH, $R^3$ = Fluor, $R^4$ = Chlor und A = CH) mit Piperazin das bekannte Bakterizid Ciprofloxacin (DE-OS 3 142 854).

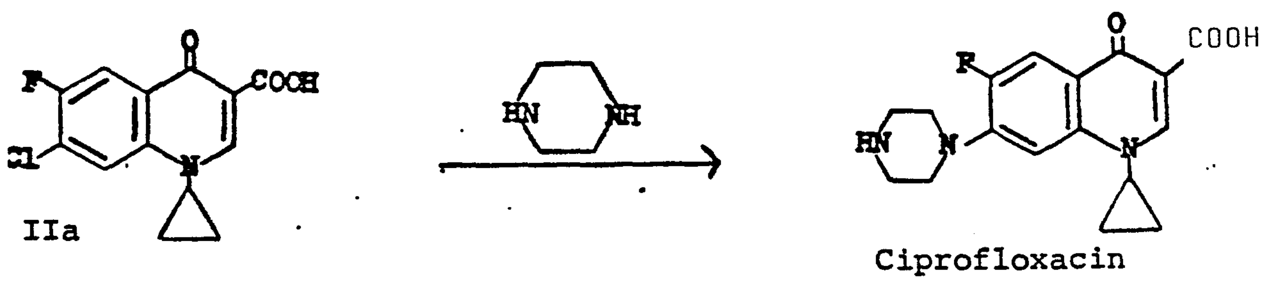

IIa

Ciprofloxacin

Die als Ausgangsstoffe für diesen Syntheseweg verwendeten Aminoacrylsäurederivate III werden wie folgt erhalten:

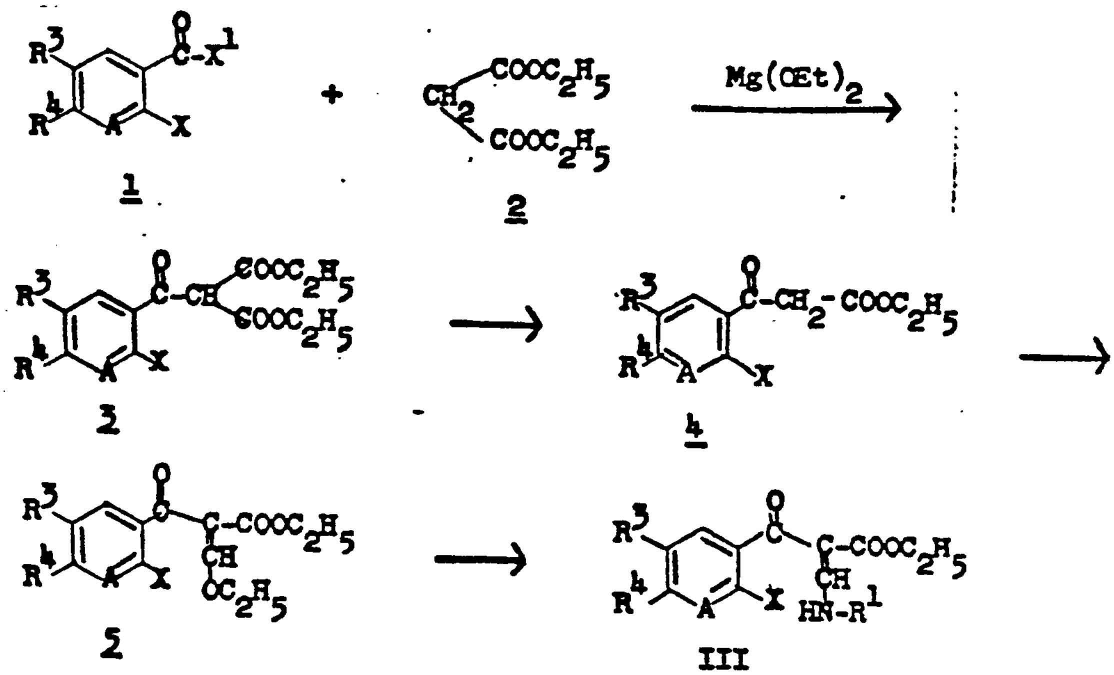


1 2 3 4 5 III

Danach wird Malonsäurediethylester (2) in Gegenwart von Magnesiumethylat mit den entsprechenden aromatischen Carbonsäure-chloriden oder -fluoriden (1) zu den Aroylmalonestern (3) acyliert (Organicum, 3. Auflage 1964, S. 438). Durch partielle Verseifung und Decarboxylierung von (3) in wäßrigem Medium mit katalytischen Mengen Schwefelsäure oder p-Toluolsulfonsäure erhält man in guter Ausbeute die Aroylessigsäureethylester (4), die mit Orthoameisensäure-triethylester/Acetanhydrid in die 2-Aroyl- bzw. 2-Hetaroyl-3-ethoxy-acryl-säureethylester (5) übergehen.

Die Umsetzung von (5) mit den Aminen $R^1NH_2$ in einem Lösungsmittel, wie z.B. Methylenchlorid, Alkohol, Chloroform, Cyclohexan oder Toluol führt in leicht exothermen Reaktionen zu den gewünschten Zwischenprodukten (III).

Die als Ausgangsstoffe verwendeten Aroylhalogenide und Hetaroylhalogenide sind bekannt oder können nach literaturbekannten Verfahren erhalten werden.

Als Beispiele seien genannt: 2,4-Dinitro-5-chlorbenzoylchlorid, 2-Nitrobenzoylchlorid, 2,6-Dinitrobenzoylchlorid, 2,4-Dinitro-benzoylchlorid, 4-Chlor-2-nitro-benzoylchlorid, 2-Methoxy-benzoylchlorid, 2-Methylmercaptobenzoylchlorid, 2-Methoxy-nicotinsäurechlorid, 4-Chlor-2-methoxybenzoylchlorid, 3,5-Dichlor-2-methoxybenzoylchlorid, 2-Methylsulfonyl-benzoylchlorid, 5-Nitro-2-methoxybenzoylchlorid, 4,5-Dichlor-2-nitro-benzoylchlorid.

Außer den in den Beispielen aufgeführten erfindungsgemäß erhältlichen Verbindungen seien noch folgende im einzelnen genannt: 6,7-Dichlor-1-cyclopropyl-1,4-dihydro-4-oxo-3-chinolincarbonsäure, 6,7-Dichlor-1-ethyl-1,4-dihydro-4-oxo-3-chinolincarbonsäure, 7-Chlor-1-cyclopropyl-6-fluor-1,4-dihydro-4-oxo-3-chinolincarbonsäure, 7-Chlor-6-fluor-1-ethyl-1,4-dihydro-4-oxo-3-chinolincarbonsäure, 1-Cyclopropyl-6,7-difluor-1,4-dihydro-4-oxo-3-chinolincarbonsäure, 6,7-Difluor-1-ethyl-1,4-dihydro-4-oxo-3-chinolincarbonsäure, 7-Chlor-1-cyclopropyl-6-nitro-1,4-dihydro-4-oxo-3-chinolincarbonsäure.

In der nachstehenden Tabelle sind die MHK-Werte einiger der erfindungsgemäßen Verbindungen angegeben.

Tabelle

| Stamm | Minimale Hemmkonzentration mcg/ml x) | | | |
|---|---|---|---|---|
| | Beispiel Nr.7 | Beispiel Nr. 6 | Beispiel Nr. 4 | Beispiel Nr. 1 |
| E. coli Neum. | 0,125 | 32 | 32 | 32 |
| E. coli T 7 | 0,125 | 32 | 64 | 64 |
| Klebsiella pneum. 63 | 0,5 | 128 | >128 | >128 |
| Klebsiella pneum. 8085 | 0,06 | 128 | 64 | 32 |
| Proteus vulg. 1017 | 0,25 | 64 | 128 | >128 |
| Proteus morg. 932 | 0,25 | 64 | 128 | >128 |
| Staphylococcus aur. 133 | 32 | >128 | >128 | >128 |

x) Agarverdünnungstest
Isosensitestmedium/Denley Multipoint - Inokulator

Beispiel 1

6-Chlor-1-cyclopropyl-7-nitro-1,4-dihydro-4-oxo-3-chinolincarbonsäure

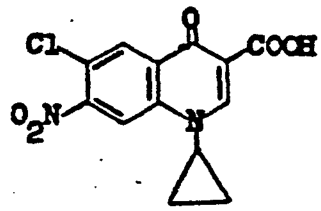

3,7 g Magnesiumspäne werden in 10 ml Ethanol und 1 ml Tetrachlormethan verrührt und nach begonnener Reaktion bei 50-60°C tropfenweise mit 24,6 g Malonsäurediethylester in 20 ml Ethanol und 80 ml Toluol versetzt. Man rührt 1 Stunde bei dieser Temperatur nach und tropft diese Lösung bei -5 bis -10°C zu einer Suspension von 40,8 g 5-Chlor-2,4-dinitrobenzoylchlorid in 50 ml Toluol. Man rührt noch 1 Stunde bei 0°C und läßt über Nacht bei Raumtemperatur stehen. Dann wird mit 80 ml Eiswasser und 10 ml konzentrierter Schwefelsäure versetzt. Die organische Phase wird abgetrennt, die wäßrige Phase mit 2 mal 50 ml Toluol extrahiert, die vereinigten organischen Phasen mit gesättigter Natriumchlorid-Lösung gewaschen, mit Natriumsulfat getrocknet und eingeengt. Der Rückstand (61,6 g) wird mit 100 ml Wasser versetzt (vorteilhaft ist hier die Zugabe von 0,3 g 4-Toluolsulfonsäure) und zur Hydrolyse und Decarboxylierung 3 Stun-

0168733

den unter Rückfluß erhitzt. Man saugt kalt ab und wäscht mit Petrolether nach. Es werden 47,5 g (5-Chlor-2,4-dinitro-benzoyl)-essigsäureethylester vom Schmelzpunkt 105-110°C erhalten. (Flüssige Aroylessigester werden in Methylenchlorid aufgenommen, mit gesättigter Kochsalzlösung gewaschen, das Lösungsmittel nach Trocknen mit $Na_2SO_4$ abdestilliert und der rohe Aroylessigester gegebenenfalls i.Hochvak. destilliert).

47,5 g des erhaltenen Esters und 33,3 g Orthoameisensäure-triethylester werden mit 39 g Essigsäureanhydrid 3 Stunden auf 150-160°C erhitzt und anschließend bei 120-130°C unter Normaldruck, danach im Hochvakuum eingeengt. Man erhält 48,2 g 2-(5-Chlor-2, 4-dinitro-benzoyl)-3-ethoxy-acrylsäure-ethylester als Öl.

48,2 g dieser Verbindung werden in 150 ml Ethanol unter Eiskühlung tropfenweise mit 7,4 g Cyclopropylamin versetzt und 1 Stunde bei Raumtemperatur gerührt. Danach wird mit 150 ml Wasser verrührt, mit Eis gekühlt, der ausgefallene Niederschlag abgesaugt, mit einem Ethanol-Wasser-Gemisch (1:1) gewaschen und getrocknet. Es werden 47,9 g 2-(5-Chlor-2,4-dinitro-benzoyl)-3-cyclopropylamino-acrylsäureethylester vom Schmelzpunkt 100-115°C erhalten. Nach der Umkristallisation aus Essigsäureethylester schmelzen die gelben Kristalle bei 130-132°C (31,4 g). Nach dem $^1$H-NMR-Spektrum liegt ein cis-trans-Gemisch vor.

3,8 g dieser Verbindung werden in 25 ml wasserfreiem Dioxan portionsweise mit 0,3 g 80 %igem Natriumhydrid

versetzt und 2 Stunden unter Rückfluß zum Sieden erhitzt. Das Reaktionsgemisch wird vorsichtig auf Eis gegossen, der Niederschlag abgesaugt, mit Wasser gewaschen und getrocknet. Nach Umkristallisation aus Ethanol werden 2 g 6-Chlor-1-cyclopropyl-7-nitro-1,4-dihydro-4-oxo-3-chinolincarbonsäureethylester vom Schmelzpunkt 208-210°C erhalten.

1,7 g des Chinoloncarbonsäureesters werden in 10 ml Eisessig und 8,5 ml Wasser mit 1,1 ml konzentrierter Schwefelsäure versetzt und 2 Stunden auf 150°C erhitzt. Das Reaktionsgemisch wird in 20 ml Eiswasser eingerührt, der Niederschlag abgesaugt, mit Wasser gewaschen und getrocknet. Es werden nach Umkristallisation aus Acetonitril 1,2 g 6-Chlor-1-cyclopropyl-7-nitro-1,4-dihydro-4-oxo-3-chinolincarbonsäure mit einem Schmelzpunkt von 248-250°C isoliert.

Beispiel 2

1-Cyclopropyl-1,4-dihydro-4-oxo-3-chinolincarbonsäure

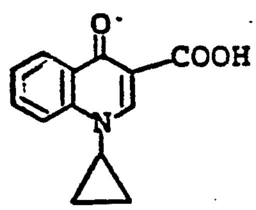

Man setzt 2-Nitrobenzoylchlorid analog Beispiel 1 um, wobei folgende Stufen durchlaufen werden:

(2-Nitro-benzoyl)-essigsäureethylester (Ausbeute: 70 %, Öl), 2-(2-Nitro-benzoyl)-3-ethoxy-acrylsäureethylester (Rohausbeute: 96 %, Öl), 2-(2-Nitro-benzoyl)-3-cyclopropylamino-acrylsäureethylester (Ausbeute: 85 %, Schmelzpunkt 144-145°C), 1-Cyclopropyl-1,4-dihydro-4-oxo-3-chinolincarbonsäureethylester (Ausbeute: 80 %, Schmelzpunkt 165°C), 1-Cyclopropyl-1,4-dihydro-4-oxo-3-chinolincarbonsäure (Ausbeute: 95 %, Schmelzpunkt 280-282°C (Zersetzung).

Die Cyclisierung des 2-(2-Nitro-benzoyl)-3-cyclopropylamino-acrylsäureethylesters zum 1-Cyclopropyl-1,4-dihydro-4-oxo-3-chinolincarbonsäureethylester kann auch mit Kaliumcarbonat/N,N-Dimethylformamid erfolgen:

3 g 2-(2-Nitro-benzoyl)-3-cyclopropylamino-acrylsäureethylester werden mit 1,4 g $K_2CO_3$ in 15 ml DMF 2 Stunden unter Rückfluß zum Sieden erhitzt. Die noch warme Suspension wird auf Eis gegossen, der Niederschlag abgesaugt, mit Wasser gewaschen und über Calciumchlorid getrocknet. Es werden 2,2 g 1-Cyclopropyl-1,4-dihydro-4-oxo-3-chinolincarbonsäureethylester vom Schmelzpunkt 163-164°C erhalten.

Beispiel 3

1-Methyl-1,4-dihydro-4-oxo-3-chinolincarbonsäure

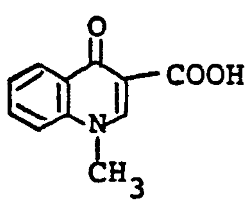

0168733

Man setzt 2-(2-Nitrobenzoyl)-3-ethoxy-acrylsäureethylester aus Beispiel 2 analog Beispiel 1 mit Methylamin um, wobei folgende Stufen durchlaufen werden:

2-(2-Nitro-benzoyl)-3-methylamino-acrylsäureethylester (Ausbeute: 80 %, Schmelzpunkt 127-129°C), 1-Methyl-1,4-dihydro-4-oxo-3-chinolincarbonsäureethylester (Ausbeute: 75 %, Schmelzpunkt 110°C), 1-Methyl-1,4-dihydro-4-oxo-3-chinolincarbonsäure (Ausbeute: 90 %, Schmelzpunkt 284-286°C (Zersetzung)).

Beispiel 4

1-Dimethylamino-1,4-dihydro-4-oxo-3-chinolincarbonsäure

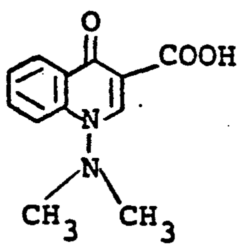

Man setzt 2-(2-Nitro-benzoyl)-3-ethoxy-acrylsäureethylester aus Beispiel 2 analog Beispiel 1 mit 1,1-Dimethylhydrazin um, wobei anschließend folgende Stufen durchlaufen werden:

2-(2-Nitro-benzoyl)-3-dimethylhydrazino-acrylsäureethylester (Ausbeute: 85 %, Schmelzpunkt 110-112°C), 1-Dimethylamino-1,4-dihydro-4-oxo-3-chinolincarbonsäure-

ethylester (Ausbeute: 77 %, Schmelzpunkt 124-126°C), 1-Dimethylamino-1,4-dihydro-4-oxo-3-chinolincarbonsäure (Ausbeute: 90 %, Schmelzpunkt 206-208°C).

Beispiel 5

1-Ethyl-1,4-dihydro-4-oxo-3-chinolincarbonsäure

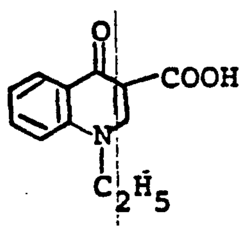

Man setzt 2-(2-Nitro-benzoyl)-3-ethoxy-acrylsäureethylester aus Beispiel 2 analog Beispiel 1 mit wäßrigem Ethylamin um, wobei folgende Stufen durchlaufen werden:

2-(2-Nitro-benzoyl)-3-ethylamino-acrylsäureethylester (Ausbeute: 80 %, Schmelzpunkt 116-118°C), 1-Ethyl-1,4-dihydro-4-oxo-3-chinolincarbonsäureethylester (Ausbeute: 95 %, Öl), 1-Ethyl-1,4-dihydro-4-oxo-3-chinolincarbonsäure (Ausbeute: 85 %, Schmelzpunkt 250-252°C).

Beispiel 6

1-Cyclopropyl-5-nitro-1,4-dihydro-4-oxo-3-chinolincarbonsäure

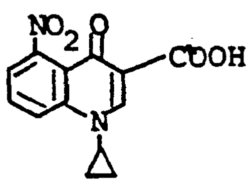

Man setzt 2,6-Dinitrobenzoylchlorid analog Beispiel 1 um, wobei folgende Stufen durchlaufen werden:

(2,6-Dinitro-benzoyl)-essigsäureethylester (Ausbeute: 80 %, Öl), 2-(2,6-Dinitro-benzoyl-3-ethoxy-acrylsäure-ethylester (Ausbeute: 95 %, Öl), 2-(2,6-Dinitrobenzoyl)-3-cyclopropylaminoacrylsäureethylester (Ausbeute: 76 %, Schmelzpunkt 143-144°C), 1-Cyclopropyl-5-nitro-1,4-dihydro-4-oxo-3-chinolincarbonsäureethylester (Ausbeute: 90 %, Schmelzpunkt 285-287°C (Zersetzung)), 1-Cyclopropyl-5-nitro-1,4-dihydro-4-oxo-3-chinolincarbonsäure (Ausbeute: 92 %, Schmelzpunkt 295-297°C (Zersetzung)).

Beispiel 7

1-Cyclopropyl-7-nitro-1,4-dihydro-4-oxo-3-chinolincarbonsäure

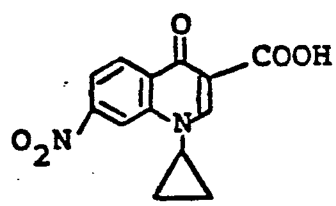

Man setzt 2,4-Dinitro-benzoylchlorid analog Beispiel 1 um, wobei folgende Stufen durchlaufen werden:

(2,4-Dinitro-benzoyl)-essigsäureethylester (Ausbeute: 75 %, Öl), 2-(2,4-Dinitro-benzoyl)-3-ethoxy-acrylsäureethylester (Ausbeute: 90 %, Öl), 2-(2,4-Dinitro-benzoyl)-3-cyclopropylamino-acrylsäureethylester (Ausbeute: 78 %,

Schmelzpunkt 129-130°C), 1-Cyclopropyl-7-nitro-1,4-dihydro-4-oxo-3-chinolincarbonsäureethylester (Ausbeute: 74 %, Schmelzpunkt 221-223°C), 1-Cyclopropyl-7-nitro-1,4-dihydro-4-oxo-3-chinolincarbonsäure (Ausbeute: 92 %, Schmelzpunkt 315-317°C (Zersetzung)).

Beispiel 8

1-Cyclopropyl-1,4-dihydro-4-oxo-3-chinolincarbonsäureethylester

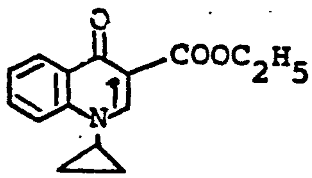

Man setzt 2-Methoxybenzoylchlorid analog Beispiel 1 um, wobei folgende Stufen durchlaufen werden:

(2-Methoxy-benzoyl)-essigsäureethylester (Ausbeute: 45 %, Siedepunkt 135-140°C/0,06 mbar), 2-(2-Methoxy-benzoyl)-3-ethoxy-acrylsäureethylester (Ausbeute: 90 %, Öl), 2-(2-Methoxy-benzoyl)-3-cyclopropylaminoacrylsäureethylester (Ausbeute: 95 %, Öl), 1-Cyclopropyl-1,4-dihydro-4-oxo-3-chinolincarbonsäureethylester (Ausbeute: 60 % nach Cyclokondensation mit $K_2CO_3$/DMF analog Beispiel 2, Schmelzpunkt 165-168°C).

Beispiel 9

1-Cyclopropyl-1,4-dihydro-4-oxo-3-chinolincarbonsäureethylester

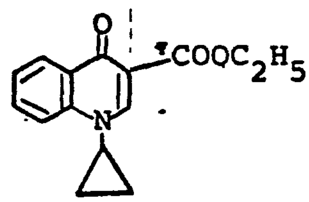

Man setzt 1-Methylmercapto-benzoylchlorid analog Beispiel 1 um, wobei folgende Stufen durchlaufen werden:

(2-Methylmercapto-benzoyl)-essigsäureethylester (Ausbeute: 65 %, Siedepunkt 157-160°C/0,07-0,1 mbar), 2-(2-Methylmercapto-benzoyl)-3-ethoxyacrylsäureethylester (Ausbeute: 95 %, Öl), 2-(2-Methylmercapto-benzoyl)-3-cyclopropylamino-acrylsäureethylester (Ausbeute: 82 %, Schmelzpunkt 97-99°C), 1-Cyclopropyl-1,4-dihydro-4-oxo-3-chinolincarbonsäureethylester (Ausbeute: 76 % nach Cyclokondensation mit $K_2CO_3$/DMF analog Beispiel 2, Schmelzpunkt 165-167°C).

Beispiel 10

1-Cyclopropyl-1,4-dihydro-4-oxo-3-chinolincarbonsäure-ethylester

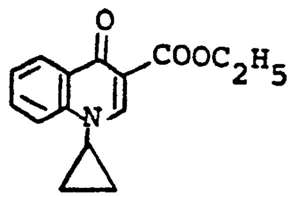

Man setzt 2-Methylsulfonyl-benzoylchlorid analog Beispiel 1 um, wobei folgende Stufen durchlaufen werden:

(2-Methylsulfonyl-benzoyl)-essigsäureethylester (Ausbeute: 55 %, Öl), 2-(2-Methylsulfonyl-benzoyl)-3-ethoxy-acrylsäureethylester (Ausbeute: 94 %, Öl), 2-(2-Methylsulfonyl-benzoyl)-3-cyclopropylaminoacrylsäureethylester (Ausbeute: 78 %, Schmelzpunkt 107°C), 1-Cyclopropyl-1,4-dihydro-4-oxo-3-chinolincarbonsäureethylester (Ausbeute: 80 %, Schmelzpunkt 164-167°C).

Patentansprüche

1. Verfahren zur Herstellung von Chinolin- bzw. Naphthyridoncarbonsäuren, dadurch gekennzeichnet, daß man Aminoacrylsäurederivate der Formel III

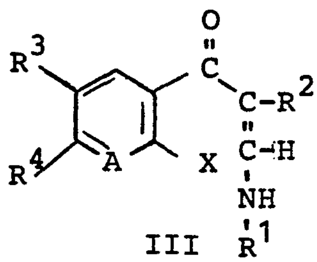

III

in der

X die Nitrogruppe, eine Alkoxy-, Alkylmercapto- oder Alkylsulfonylgruppe mit jeweils bis zu drei Kohlenstoffatomen sein kann,

$R^1$ $C_1$-$C_3$-Alkyl, Cyclopropyl, Phenyl, die Amino-, Methylamino oder Dimethylaminogruppe darstellt,

$R^2$ eine Alkoxycarbonylgruppe mit 1-3 Kohlenstoffatomen im Alkoholteil bedeutet,

$R^3$ Wasserstoff, Nitro oder Halogen, insbesondere Fluor oder Chlor, bedeutet,

$R^4$ Wasserstoff oder Halogen, insbesondere Fluor oder Chlor, darstellt, und

A Stickstoff oder ein gegebenenfalls substituiertes Kohlenstoffatom-C-$R^5$ sein kann, wobei

$R^5$ **Wasserstoff, Fluor oder Chlor bedeutet,**

zu Verbindungen der Formel II

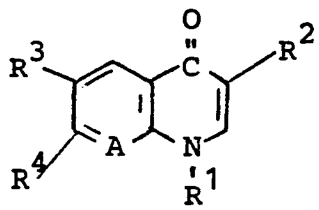

II

cyclokondensiert, und, die Alkoxycarbonylgruppe $R^2$ verseift zu Verbindungen der Formel I

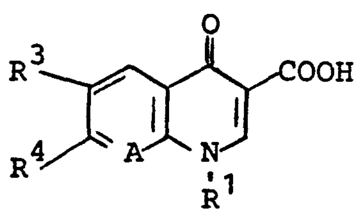

2. Verfahren nach Anspruch 1, dadurch gekennzeichnet, daß man als Kondensationsmittel für die Cyclokondensierung Natrium- oder Kaliumcarbonat verwendet.

3. Verfahren nach Anspruch 1, dadurch gekennzeichnet, daß man als Kondensationsmittel für die Cyclokondensierung Natrium- oder Kaliumalkoholate mit 1-4 C-Atomen im Alkoholrest verwendet.

0168733

4. Verfahren nach Anspruch 1, dadurch gekennzeichnet, daß man als Kondensationsmittel für die Cyclokondensierung Natriumhydrid verwendet.

5. Verfahren nach Anspruch 1, dadurch gekennzeichnet, daß man die Cyclokondensation bei Temperaturen von 60-300°C, vorzugsweise 80-180°C durchführt.

6. Verfahren nach Anspruch 1, dadurch gekennzeichnet, daß man die Cyclokondensation in einem Verdünnungsmittel durchführt.

7. Verfahren nach Anspruch 6, dadurch gekennzeichnet, daß man Dioxan, Dimethylsulfoxid, N-Methylpyrrolidon, Sulfolan oder N,N-Dimethylformamid als Verdünnungsmittel verwendet.

8. Verfahren nach Anspruch 1, dadurch gekennzeichnet, daß man die Cyclokondensation bei Temperaturen von 80 - 180°C durchführt.

9. Verfahren nach Anspruch 1, dadurch gekennzeichnet, daß man die Alkoxycarbonylgruppe $R^2$ unter sauren Bedingungen verseift.

10. Verfahren nach Anspruch 1, dadurch gekennzeichnet, daß man 2,4-Dinitro-5-chlorbenzoylchlorid, 2-Nitrobenzoylchlorid, 2,6-Dinitrobenzoylchlorid, 2,4-Dinitrobenzoylchlorid, 4-Chlor-2-nitro-benzoylchlorid, 2-Methoxy-benzoylchlorid, 2-Methylmercaptobenzoylchlorid, 2-Methoxy-nicotinsäurechlorid, 4-Chlor-2-methoxy-benzoylchlorid, 3,5-Dichlor-2-methoxybenzoylchlorid, 2-Methylsulfonyl-benzoylchlorid, 5-Nitro-2-methoxybenzoylchlorid, 4,5-Dichlor-2-nitro-

benzoylchlorid als Ausgangsverbindungen für die Synthese der Verbindungen der Formel III verwendet.